# EUROPÄISCHE PATENTSCHRIFT

(11) **EP 3 497 141 B1**
(45) Veröffentlichungstag und Bekanntmachung des Hinweises auf die Patenterteilung: **25.11.2020**
(21) Anmeldenummer: 17743368.7
(22) Anmeldetag: 01.08.2017
(51) Int. Cl.: C08F 220/06, A61L 15/60, C08J 3/12, C08J 3/24, C08K 5/00

(54) **VERFAHREN ZUR HERSTELLUNG VON SUPERABSORBERN**
METHOD FOR PRODUCING SUPERABSORBENT
PROCÉDÉ DE PRODUCTION DE SUPERABSORBANT

(30) Priorität: 10.08.2016 EP 16183579
(43) Veröffentlichungstag der Anmeldung: 19.06.2019
(73) Patentinhaber: BASF SE, 67056 Ludwigshafen am Rhein (DE)
(72) Erfinder: HARTNAGEL, Kristine, 67056 Ludwigshafen (DE); GIEGER, Thomas, 67056 Ludwigshafen (DE); DANIEL, Thomas, 67056 Ludwigshafen (DE); SCHROEDER, Marcus, 67056 Ludwigshafen (DE); MORANO, Michelle, Charlotte, NC 28273 (US); GREENE, Nathaniel Troy, Charlotte, NC 28273 (US)
(74) Vertreter: BASF IP Association
(86) Internationale Anmeldenummer: PCT/EP2017/069446
(87) Internationale Veröffentlichungsnummer: WO 2018/029045

(56) Entgegenhaltungen:
- EP-A1- 2 163 302
- WO-A1-2013/144026
- WO-A1-2013/144027

## Beschreibung

Die vorliegende Erfindung betrifft ein Verfahren zur Herstellung von Superabsorbern, umfassend Polymerisation einer Monomerlösung und thermischer Oberflächennachvernetzung, wobei die Monomerlösung mindestens 0,75 Gew.-% einer Hydroxyphosphonsäure, berechnet auf die Gesamtmenge an eingesetztem Monomer, enthält und den Polymerpartikeln vor, während oder nach der thermischen Oberflächennachvernetzung mindestens 0,09 Gew.-% Aluminiumkationen, berechnet auf die Gesamtmenge an eingesetzten Polymerpartikeln, zugesetzt werden.

Superabsorber werden zur Herstellung von Windeln, Tampons, Damenbinden und anderen Hygieneartikeln, aber auch als wasserzurückhaltende Mittel im landwirtschaftlichen Gartenbau verwendet. Die Superabsorber werden auch als wasserabsorbierende Polymere bezeichnet.

Die Herstellung von Superabsorbern wird in der Monographie "Modern Superabsorbent Polymer Technology", F.L. Buchholz und A.T. Graham, Wiley-VCH, 1998, Seiten 71 bis 103, beschrieben.

Die Eigenschaften der Superabsorber können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm² (AUL0.3psi) durchläuft ein Maximum.

Zur Verbesserung der Anwendungseigenschaften, wie beispielsweise Gelbettpermeabilität (GBP) und Absorption unter einem Druck von 49,2 g/cm² (AUL0.7psi), werden Superabsorberpartikel im allgemeinen oberflächennachvernetzt. Dadurch steigt der Vernetzungsgrad der Partikeloberfläche, wodurch die Absorption unter einem Druck von 49,2 g/cm² (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet und thermisch oberflächennachvernetzt. Dazu geeignete Vernetzer sind Verbindungen, die mit mindestens zwei Carboxylatgruppen der Polymerpartikel kovalente Bindungen bilden können.

WO 2011/113777 A1 beschreibt die Herstellung von Superabsorbern. Dabei werden während der Polymerisation oder später eine anorganische Phosphorsäure oder deren Salz und eine organische 2-Hydroxysäure oder deren Salz zugesetzt.

WO 2013/144026 A1 beschreibt Superabsorber, deren Oberflächen mit mehrwertigen Metallionen komplexiert sind und ein Phosphonsäurederivat enthalten.

Aufgabe der vorliegenden Erfindung war die Bereitstellung eines verbesserten Verfahrens zur Herstellung farbstabiler Superabsorber mit hoher Gelbettpermeabilität (GBP).

Gelöst wurde die Aufgabe durch ein Verfahren zur Herstellung von Superabsorbern durch Polymerisation einer Monomerlösung oder -suspension, enthaltend
a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
   umfassend die Schritte
   i) Polymerisation der Monomerlösung zu einem Polymergel,
   ii) optional Zerkleinerung des erhaltenen Polymergels,
   iii) Trocknung des Polymergels,
   iv) Mahlung und Klassierung des getrockneten Polymergels zu Polymerpartikeln,
   v) thermischer Oberflächennachvernetzung der klassierten Polymerpartikel,
   vi) optional erneute Klassierung der oberflächennachvernetzten Polymerpartikel,
   vii) optional Rückführung in Schritt iv) abgetrennter Polymerpartikel vor Schritt iii) und
   viii) optional Rückführung in Schritt vi) abgetrennter Polymerpartikel vor Schritt iii),
   dadurch gekennzeichnet, dass der Monomerlösung vor Schritt i) mindestens 0,75 Gew.-% einer Hydroxyphosphonsäure oder deren Salze, berechnet auf die Gesamtmenge an eingesetztem Monomer a), zugesetzt werden, dass den Polymerpartikeln zwischen Schritt iv) und Schritt vi) mindestens 0,09 Gew.-% Aluminiumkationen, berechnet auf die Gesamtmenge an eingesetzten Polymerpartikeln, zugesetzt werden und dass das Aluminiumkation als Aluminiumsulfat eingesetzt wird.

Als Salze der Hydroxyphosphonsäure werden vorzugsweise Alkalimetallsalze, besonders bevorzugt Natrium- und Kaliumsalze, ganz besonders bevorzugt Natriumsalze, eingesetzt. Die neutralisierbaren Protonen der Hydroxyphosphonsäure können dabei ganz oder teilweise durch beliebige Alkalimetallkationen ersetzt werden.

In einer bevorzugte Ausführungsform der vorliegenden Erfindung werden die oberflächennachvernetzten Polymerpartikel in Schritt vi) erneut klassiert und in Schritt vi) abgetrennte Polymerpartikel vor Schritt iii) rückgeführt. Die rückgeführten Polymerpartikel weisen eine Partikelgröße von vorzugsweise weniger als 250 µm, besonders bevorzugt von weniger als 200 µm, ganz besonders bevorzugt von weniger als 150 µm, auf.

Vorzugsweise wird 1-Hydroxyethyliden-1,1'-diphosphonsäure als Hydroxyphosphonsäure eingesetzt.

In einer bevorzugten Ausführungsform der vorliegenden Erfindung werden Aluminiumkationen vor Schritt v) zugesetzt.

Der Monomerlösung werden vorzugsweise mindestens 0,80 Gew.-%, besonders bevorzugt mindesten 0,85 Gew.-%, ganz besonders bevorzugt mindestens 0,90 Gew.-%, einer Hydroxyphosphonsäure oder deren Salze, berechnet auf die Gesamtmenge an eingesetztem Monomer a), zugesetzt.

Den Polymerpartikeln werden zwischen Schritt iv) und Schritt vi) vorzugsweise mindestens 0,10 Gew.-%, besonders bevorzugt mindestens 0,11 Gew.-%, ganz besonders bevorzugt mindestens 0,12 Gew.-%, Aluminiumkationen, berechnet auf die Gesamtmenge an eingesetzten Polymerpartikeln, zugesetzt.

Der vorliegenden Erfindung liegt die Erkenntnis zugrunde, dass eine hohe Farbstabilität und eine hohe Gelbettpermeabilität (GBP) nur mit einer relativ großen Menge an Hydroxyphosphonsäure in der Monomerlösung und einer relativ großen Menge an Aluminiumkationen auf der Partikeloberfläche erreicht werden. Die Verwendung von Sulfat als Gegenion führt zu einer deutlich erhöhten Gelbettpermeabilität (GBP).

Weiterhin wurde gefunden, dass rückgeführte oberflächennachvernetzte Superabsorber keine Hydroxyphosphonsäure aus der Monomerlösung aufnehmen. Werden solche Superabsorber ("fines") in das Verfahren rückgeführt, so sind diese Polymerpartikel nicht farbstabil und führen zu punktuellen Verfärbungen. Derartige punktuelle Verfärbungen werden als besonders störend empfunden. Überraschenderweise wird dies auch beobachtet, wenn die rückgeführten Superabsorber auf der Stufe der Polymerpartikel mit einer Hydroxyphosphonsäure beschichtet wurden. Möglicherweise werden dabei einige, insbesondere die kleineren Polymerpartikel, nur unzureichend oder gar nicht mit der Beschichtungslösung benetzt.

Im Folgenden wird die Herstellung der Superabsorber näher erläutert:
Die Superabsorber werden in Schritt i) durch Polymerisation einer Monomerlösung oder -suspension hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 02/055469 A1, der WO 03/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomers a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 03/104299 A1, WO 03/104300 A1, WO 03/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 02/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 03/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,25 bis 1,5 Gew.-%, besonders bevorzugt 0,3 bis 1,2 Gew.-%, ganz besonders bevorzugt 0,4 bis 0,8 Gew.-%, jeweils berechnet auf die Gesamtmenge an eingesetztem Monomer a). Mit steigendem Vernetzergehalt sinkt die Zentrifugenretentionskapazität (CRC) und die Absorption unter einem Druck von 21,0 g/cm² durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird vorzugsweise das Dinatriumsalz der 2-Hydroxy-2-sulfonatoessigsäure oder ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit die Löslichkeit überschreitendem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren für die Polymerisation in Schritt i) sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Schritt ii) zerkleinert werden muss, beispielsweise in einem Extruder oder Kneter.

Zur Verbesserung der Trocknungseigenschaften kann das mittels eines Kneters erhaltene zerkleinerte Polymergel zusätzlich in Schritt ii) extrudiert werden.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 85 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen. Feste Carbonate und Hydrogencarbonate können hierbei auch in verkapselter Form eingesetzt werden, vorzugsweise in die Monomerlösung direkt vor der Polymerisation, während oder nach der Polymerisation ins Polymergel und vor dessen Trocknung. Die Verkapselung erfolgt durch Beschichtung der Oberfläche mit einem unlöslichen oder nur langsam löslichen Material (beispielsweise mittels filmbildender Polymere, inerter anorganischer Materialien oder schmelzbaren organischer Materialien), welches die Lösung und Reaktion des festen Carbonats oder Hydrogencarbonats so verzögert, dass erst während der Trocknung Kohlendioxid freigesetzt wird und der entstehende Superabsorber eine hohe innere Porosität aufweist.

Optional kann zur Monomerlösung vor oder während der Polymerisation ein Tensid zugegeben werden und die Monomerlösung dann vor oder während der Polymerisation mit einem Inertgas oder Wasserdampf oder durch starkes Rühren geschäumt werden. Das Tensid kann anionisch, kationisch, zwitterionisch oder auch nicht-ionisch sein. Bevorzugt wird ein hautfreundliches Tensid eingesetzt.

Das Polymergel wird dann in Schritt iii) mit einem Umluftbandtrockner getrocknet bis der Restfeuchtegehalt vorzugsweise 0,5 bis 10 Gew.-%, besonders bevorzugt 1 bis 6 Gew.-%, ganz besonders bevorzugt 1,5 bis 4 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur T_{g} auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Polymergels beträgt vor der Trocknung vorzugsweise von 25 und 90 Gew.-%, besonders bevorzugt von 35 bis 70 Gew.-%, ganz besonders bevorzugt von 40 bis 60 Gew.-%. Anschließend wird das getrocknete Polymergel gebrochen und optional grob zerkleinert.

Das getrocknete Polymergel wird hiernach in Schritt iv) gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion Schritt xii) abgetrennten Polymerpartikel beträgt vorzugsweise von 150 bis 850 µm, besonders bevorzugt von 250 bis 600 µm, ganz besonders von 300 bis 500 µm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Polymerpartikeln mit einer Partikelgröße von größer 150 µm beträgt vorzugsweise mindestens 90 Gew.-%, besonders bevorzugt mindestens 95 Gew.-%, ganz besonders bevorzugt mindestens 98 Gew.-%.

Polymerpartikel mit zu niedriger Partikelgröße senken die Gelbettpermeabilität (GBP). Daher sollte der Anteil zu kleiner Polymerpartikel ("fines") niedrig sein.

Zu kleine Polymerpartikel werden daher üblicherweise abgetrennt und in das Verfahren rückgeführt, vorzugsweise vor, während oder unmittelbar nach der Polymerisation in Schritt i), d.h. vor der Trocknung des Polymergels in Schritt iii). Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung in Schritt v) oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation in Schritt i) zugesetzt. Es ist aber auch möglich die zu kleinen Polymerpartikel in einem dem Polymerisationsreaktor nachgeschalteten Schritt ii), beispielsweise in einem Kneter oder Extruder, in das Polymergel einzuarbeiten.

Werden die zu kleinen Polymerpartikel sehr früh zugesetzt, beispielsweise bereits zur Monomerlösung, so wird dadurch die Zentrifugenretentionskapazität (CRC) der erhaltenen Polymerpartikel gesenkt. Dies kann aber beispielsweise durch Anpassung der Einsatzmenge an Vernetzer b) kompensiert werden.

Der Anteil an Polymerpartikeln mit einer Partikelgröße von höchstens 850 µm, beträgt vorzugsweise mindestens 90 Gew.-%, besonders bevorzugt mindestens 95 Gew.-%, ganz besonders bevorzugt mindestens 98 Gew.-%.

Der Anteil an Polymerpartikeln mit einer Partikelgröße von höchstens 600 µm, beträgt vorzugsweise mindestens 90 Gew.-%, besonders bevorzugt mindestens 95 Gew.-%, ganz besonders bevorzugt mindestens 98 Gew.-%.

Polymerpartikel mit zu großer Partikelgröße senken die Quellgeschwindigkeit. Daher sollte der Anteil zu großer Polymerpartikel ebenfalls niedrig sein. Zu große Polymerpartikel werden daher üblicherweise abgetrennt und in die Mahlung rückgeführt.

Die Polymerpartikel können zur weiteren Verbesserung der Eigenschaften in Schritt v) thermisch oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder β-Hydroxyalkylamide, wie in DE 102 04 938 A1 und US 6,239,230 beschrieben.

Des Weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 03/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 3 Gew.-%, besonders bevorzugt 0,02 bis 1 Gew.-%, ganz besonders bevorzugt 0,05 bis 0,2 Gew.-%, jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel oberflächennachvernetzt und getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und thermisch oberflächennachvernetzt.

Bevorzugte Reaktionstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die Polymerpartikel nach der Oberflächennachvernetzung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die Polymerpartikel auf vorzugsweise 40 bis 90°C, besonders bevorzugt 45 bis 80°C, ganz besonders bevorzugt 50 bis 70°C, abgekühlt.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 40 bis 120°C, besonders bevorzugt bei 50 bis 110°C, ganz besonders bevorzugt bei 60 bis 100°C, durchgeführt. Bei zu niedrigen Temperaturen neigen die Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Oberflächennachvernetzung durchgeführt.

Geeignete Beschichtungen zur Verbesserung der Quellgeschwindigkeit sowie der Gelbettpermeabilität (GBP) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20. Geeignete Beschichtungen zur Staubbindung, zur Verringerung der Verbackungsneigung sowie zur Erhöhung der mechanischen Stabilität sind Polymerdispersionen, wie in EP 0 703 265 B1 beschrieben, und Wachse, wie in US 5 840 321 beschrieben.

Ein weiterer Gegenstand der vorliegenden Erfindung sind die gemäß dem erfindungsgemäßen Verfahren hergestellten Superabsorber, wobei die Superabsorberpartikel eine Zentrifugenretentionskapazität von mindestens 25 g/g, eine Absorption unter einem Druck von 49,2 g/cm² von mindestens 15 g/g, eine Gelbettpermeabilität von mindestens 60 Darcies aufweisen und die Superabsorberpartikel nach einer Lagerung von 14 Tagen bei einer Temperatur von 70°C und einer relativen Feuchte von 80% einen L-Wert von mindestens 67, einen a-Wert von weniger als 7,0 und einen b-Wert von weniger als 14,5 aufweisen.

Die erfindungsgemäßen Superabsorberpartikel weisen auch in der Mischung mit Zellstoff, wie sie üblicherweise in Windeln vorliegt, eine hohe Farbstabilität auf. Superabsorberpartikeln, bei denen das farbstabilisierende Mittel nur auf dessen Oberfläche aufgebracht wurde, weisen dagegen in der Mischung mit Zellstoff eine deutlich geringere Farbstabiltät auf.

Die gemäß dem erfindungsgemäßen Verfahren hergestellten Superabsorberpartikel weisen eine Zentrifugenretentionskapazität (CRC) von vorzugsweise mindestens 26 g/g, besonders bevorzugt mindestens 27 g/g, ganz besonders bevorzugt mindestens 28 g/g, auf. Die Zentrifugenretentionskapazität (CRC) der Superabsorberpartikel beträgt üblicherweise weniger als 60 g/g.

Die gemäß dem erfindungsgemäßen Verfahren hergestellten Superabsorberpartikel weisen eine Absorption unter einem Druck von 49,2 g/cm² (AUL0.7psi) von vorzugsweise mindestens 16 g/g, besonders bevorzugt mindestens 17 g/g, ganz besonders bevorzugt mindestens 18 g/g, auf. Die Absorption unter einem Druck von 49,2 g/cm² (AUL0.7psi) der Superabsorberpartikel beträgt üblicherweise weniger als 35 g/g.

Die gemäß dem erfindungsgemäßen Verfahren hergestellten Superabsorberpartikel weisen eine Gelbettpermeabilität (GBP) von vorzugsweise mindestens 65 Darcies, besonders bevorzugt mindestens 70 Darcies, ganz besonders bevorzugt mindestens 75 Darcies. Die Gelbettpermeabilität (GBP) der Superabsorberpartikel beträgt üblicherweise weniger als 400 Darcies.

Die gemäß dem erfindungsgemäßen Verfahren hergestellten Superabsorberpartikel weisen nach einer Lagerung von 14 Tagen bei einer Temperatur von 70°C und einer relativen Feuchte von 80% einen L-Wert von vorzugsweise mindestens 68, besonders bevorzugt mindestens 69, ganz besonders bevorzugt mindestens 70, auf.

Die gemäß dem erfindungsgemäßen Verfahren hergestellten Superabsorberpartikel weisen nach einer Lagerung von 14 Tagen bei einer Temperatur von 70°C und einer relativen Feuchte von 80% vorzugsweise einen a-Wert von weniger als 6,6 und einen b-Wert von weniger als 14,0, besonders bevorzugt einen a-Wert von weniger als 6,2 und einen b-Wert von weniger als 13,5, ganz besonders bevorzugt einen a-Wert von weniger als 5,8 und einen b-Wert von weniger als 13,0, auf.

### Methoden:

Die nachfolgend beschriebenen, mit "WSP" bezeichneten Standard-Testmethoden werden beschrieben in: "Standard Test Methods for the Nonwovens Industry", Ausgabe 2005, gemeinsam herausgegeben von den "Worldwide Strategic Partners" EDANA (Avenue Eugene Plasky, 157, 1030 Brüssel, Belgien, www.edana.org) und INDA (1100 Crescent Green, Suite 115, Cary, North Carolina 27518, USA, www.inda.org). Diese Veröffentlichung ist sowohl von EDANA als auch von INDA erhältlich.

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymerpartikel werden vor der Messung gut durchmischt.

### Zentrifugenretentionskapazität (Centrifuge Retention Capacity)

Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

### Absorption unter einem Druck von 21,0 g/cm² (Absorption under Load)

Die Absorption unter einem Druck von 21,0 g/cm² (AUL0.3psi) der wasserabsorbierenden Polymerpartikel wird gemäß der von der EDANA empfohlenen Testmethode Nr. WSP 242.2-05 "Absorption Under Pressure, Gravimetric Determination" bestimmt.

### Absorption unter einem Druck von 49,2 g/cm² (Absorption under Load)

Die Absorption unter einem Druck von 49,2 g/cm² (AUL0.7psi) wird analog der von der EDANA empfohlenen Testmethode Nr. WSP 242.2-05 "Absorption Under Pressure, Gravimetric Determination" bestimmt, wobei statt eines Drucks von 21,0 g/cm² (AUL0.3psi) ein Druck von 49,2 g/cm² (AUL0.7psi) eingestellt wird.

### Gelbettpermeabilität (Gel Bed Permeability)

Die Gelbettpermeabilität (GBP) einer gequollenen Gelschicht unter Druckbelastung von 0,3 psi (2070 Pa) wird, wie in US 2005/0256757 beschrieben (Absätze [0061] und [0075]), als Gel-Bed-Permeability einer gequollenen Gelschicht aus wasserabsorbierenden Polymerpartikeln bestimmt.

### CIE-Farbzahl (L, a, b)

Die Farbmessung wird entsprechend dem CIELAB-Verfahren (Hunterlab, Band 8, Jahrgang 1996, Heft 7, Seiten 1 bis 4) mit einem Colorimeter, Modell "LabScan XE S/N LX17309" (HunterLab, Reston, US) durchgeführt. Dabei werden die Farben über die Koordinaten L, a und b eines dreidimensionalen Systems beschrieben. Dabei gibt L die Helligkeit an, wobei L = 0 schwarz und L = 100 weiß bedeutet. Die Werte für a und b geben die Position der Farbe auf den Farbachsen rot/grün bzw. gelb/blau an, wobei +a für rot, -a für grün, +b für gelb und -b für blau steht. Nach der Formel HC60 = L-3b wird der HC60-Wert berechnet.

Die Farbmessung entspricht dem Dreibereichsverfahren nach DIN 5033-6.

### Beispiele

### Beispiel 1

Eine für 30 Minuten mit Stickstoffgas vom Luftsauerstoff befreite Monomerlösung aus 406,8 g Acrylsäure, 4271,4 g wässriger Natriumacrylatlösung (37,3 gew.-%ig), 130,4 g Wasser und 5,86 g 3-fach ethoxiliertes Glycerintriacrylat (ca. 85 gew.-%ig) wurde in einem Polymerisationsreaktor mit zwei achsparallelen Wellen vom Typ LUK 8.0 K2 (Coperion Werner & Pfleiderer GmbH & Co. KG; Stuttgart, Deutschland) polymerisiert. Die Monomerlösung wurde zusätzlich mit 77,29 g einer wässrigen Lösung des Dinatriumsalzes der 1-Hydroxyethyliden-1,1-diphosphonsäure (20 gew.-%ig) versetzt. Die Polymerisation wurde durch Zugabe von 15,84 g wässriger Natriumperoxodisulfatlösung (15 gew.-%ig), 1,41 g wässrigem Wasserstoffperoxid (1 gew.-%ig) und 91,12 g wässriger Ascorbinsäurelösung (0,5 gew.-%ig) initiiert. Die Polymersiation war nach ca. 30 Minuten beendet. Das erhaltene Polymergel wurde dreimal mit Hilfe eines handelsüblichen Fleischwolfs mit 6 mm-Lochscheibe gewolft und im Labortrockenschrank 90 Minuten bei 175°C getrocknet. Das getrocknete Polymer wurde gemahlen und auf eine Partikelgröße von 150 bis 710 µm abgesiebt. Das so hergestellte Grundpolymer wies eine Zentrifugenretentionskapazität (CRC) von 38 g/g auf.

1000 g Grundpolymer wurden zur Oberflächennachvernetzung in einem Pflugscharmischer vom Typ M5R (Gebrüder Lödige Maschinenbau GmbH; Paderborn; Deutschland) bei 23°C und einer Wellendrehzahl von 200 Umdrehungen pro Minute mittels zweier Zweistoff-Sprühdüsen mit den folgenden Lösungen beschichtet:

| | |
|---|---|
| Lösung I: | 0,50 g N-(2-Hydroxyethyl)-2-oxazolidinon |
| | 0,50 g 1,3-Propandiol |
| | 33,10 g wässrige Aluminiumsulfatlösung (26,8 gew.-%ig) |
| Lösung II: | 10,0 g Isopropanol |

Nach dem Aufsprühen der beiden Lösungen wurde die Temperatur auf 195°C erhöht und das Reaktionsgemisch 75 Minuten bei dieser Temperatur und einer Wellendrehzahl von 60 Umdrehungen pro Minute gehalten. Anschließend wurde das Reaktionsgemisch bei 23°C erneut auf eine Partikelgröße von 150 bis 710 µm abgesiebt.

Die erhaltenen Superabsorberpartikel wurden analysiert. Zur Bestimmung der Farbstabilität wurden die Superabsorberpartikel 14 Tage bei 70°C und einer relativen Feuchte von 80% gelagert. Die Ergebnisse sind in der Tabelle 1 zusammengefasst.

### Beispiel 2 (Vergleichsbeispiel)

Es wurde verfahren wie unter Beispiel 1. Die Monomerlösung wurde statt mit 77,29 g einer wässrigen Lösung des Dinatriumsalzes der 1-Hydroxyethyliden-1,1-diphosphonsäure (20 gew.-%ig) nur mit 36,61 g einer wässrigen Lösung des Dinatriumsalzes der 1-Hydroxyethyliden-1,1-diphosphonsäure (20 gew.-%ig) versetzt.

Die erhaltenen Superabsorberpartikel wurden analysiert. Zur Bestimmung der Farbstabilität wurden die Superabsorberpartikel 14 Tage bei 70°C und einer relativen Feuchte von 80% gelagert. Die Ergebnisse sind in der Tabelle 1 zusammengefasst.

### Beispiel 3 (Vergleichsbeispiel)

Es wurde verfahren wie unter Beispiel 1. Zur Oberflächennachvernetzung wurden statt 42,90 g wässrige Aluminiumsulfatlösung (26,8 gew.-%ig) nur 11,10 g wässrige Aluminiumsulfatlösung (26,8 gew.-%ig) eingesetzt.

Die erhaltenen Superabsorberpartikel wurden analysiert. Zur Bestimmung der Farbstabilität wurden die Superabsorberpartikel 14 Tage bei 70°C und einer relativen Feuchte von 80% gelagert. Die Ergebnisse sind in der Tabelle 1 zusammengefasst.

### Beispiel 4 (Vergleichsbeispiel)

Es wurde verfahren wie unter Beispiel 1. Die Monomerlösung enthielt kein Dinatriumsalz der 1-Hydroxyethyliden-1,1-diphosphonsäure. Stattdessen wurde die Monomerlösung mit 15,46 g des Dinatriumsalz der 2-Hydroxy-2-sulfonatoessigsäure versetzt.

Während der Polymerisation wickelte sich das entstandene Polymergel um die Wellen des Polymerisationsreaktors. Der Versuch musste abgebrochen werden.

### Beispiel 5 (Vergleichsbeispiel)

Es wurde verfahren wie unter Beispiel 1. Die Monomerlösung enthielt kein Dinatriumsalz der 1-Hydroxyethyliden-1,1-diphosphonsäure.

Die erhaltenen Superabsorberpartikel wurden analysiert. Zur Bestimmung der Farbstabilität wurden die Superabsorberpartikel 14 Tage bei 70°C und einer relativen Feuchte von 80% gelagert. Die Ergebnisse sind in der Tabelle 1 zusammengefasst.

### Beispiel 6 (Vergleichsbeispiel)

Es wurde verfahren wie unter Beispiel 1. Zur Oberflächennachvernetzung wurde kein Aluminiumsulfat eingesetzt. Stattdessen wurden 80,8 g wässrige Aluminiumtrilaktatlösung (18,9 gew.-%ig) eingesetzt

Die erhaltenen Superabsorberpartikel wurden analysiert. Zur Bestimmung der Farbstabilität wurden die Superabsorberpartikel 14 Tage bei 70°C und einer relativen Feuchte von 80% gelagert. Die Ergebnisse sind in der Tabelle 1 zusammengefasst.

**Tab. 1:**

| Bsp. | Monomerlösung | SXL | CRC [g/g] | AUL0.7psi [g/g] | GBP [darcies] | L | a | b |
|---|---|---|---|---|---|---|---|---|
| 1 | 9500ppm Cublen | 1500 ppm Al³⁺ (als Sulfat) | 29,8 | 21,4 | 109 | 74 | 5,3 | 12 |
| 2*) | 4500ppm Cublen | 1500 ppm Al³⁺ (als Sulfat) | 30,4 | 21,5 | 101 | 63 | 7,3 | 15 |
| 3*) | 9500ppm Cublen | 500 ppm Al³⁺ (als Sulfat) | 31,1 | 23,3 | 26 | 85 | 0,9 | 8,6 |
| 4*) | 9500ppm Blancolen | **) | - | - | - | - | - | - |
| 5*) | kein Cublen | 1500 ppm Al³⁺ (als Sulfat) | 29,9 | 20,6 | 118 | 50 | 9,1 | 16,7 |
| 6*) | 9500ppm Cublen | 1500 ppm Al³⁺ (als Laktat) | 29,3 | 25,3 | 17 | 79 | 3,7 | 11 |

| | | | | | | | | |
|---|---|---|---|---|---|---|---|---|
| SXL: Oberflächen nachvernetzung Cublen: Dinatriumsalz der 1-Hydroxyethyliden-1,1-diphosphonsäure Blancolen: Dinatriumsalz der 2-Hydroxy-2-sulfonatoessigsäure *) Vergleichsversuch **) nicht möglich, Polymerisation abgebrochen | | | | | | | | |

Die Ergebnisse der Beispiele 1 bis 3 zeigen, dass Superabsorber mit hoher Farbstabilität und hoher Gelbettpermeabilität (GBP) nur mit großer Menge an Hydroxyphosphonsäure (Cublen) und großer Menge an Aluminiumkationen erhalten werden.

Beispiel 4 zeigt, das 2-Hydroxysulfonsäuren (Blancolen) in den für die Farbstabilisierung notwendigen Mengen in der Monomerlösung störend in die Polymerisation eingreifen.

Die Beispiele 1 und 6 zeigen die Vorteilen von Sulfat gegenüber Laktat als Gegenion.

### Beispiel 7

Durch Mischen von Wasser, wässriger Natronlauge und Acrylsäure wurde eine 43,0 gew.-%ige Acrylsäure/Natriumacrylatlösung hergestellt. Der Neutralisationsgrad der so hergestellten Monomerlösung betrug 72,0 mol-%.

Die Monomerlösung wurde bei ca. 23°C mit Stickstoff entgast. Als mehrfach ethylenisch ungesättigter Vernetzer wurde 3-fach ethoxiliertes Glyzeryintriacrylat (ca. 85gew.-%ig) verwendet.

Die Einsatzmenge an 3-fach ethoxiliertem Glyzeryintriacrylat, berechnet auf die Gesamtmenge an eingesetzter Acrylsäure, betrug 0,16 Gew.-%.

Zusätzlich wurde der Monomerlösung eine 20gew.-%ige wässrige Lösung des Dinatriumsalzes der 1-Hydroxyethyliden-1,1-diphosphonsäure zugesetzt. Die Einsatzmenge an Dinatriumsalz der 1-Hydroxyethyliden-1,1-diphosphonsäure, berechnet auf die Gesamtmenge an eingesetzter Acrylsäure, betrug 0,30 Gew.-%.

Zur Initiierung der radikalischen Polymerisation wurden folgende Komponenten eingesetzt: Wasserstoffperoxid (0,002 Gew.-% einer 1,0gew.-%igen wässrigen Lösung), Natriumperoxodisulfat (0,15 Gew.-% einer 15gew.-%igen wässrigen Lösung), sowie Ascorbinsäure (0,01 Gew.-% einer 0,5gew.-%igen wässrigen Lösung). Die Gewichtsprozente wurden auf die Gesamtmenge an eingesetzter Acrylsäure berechnet.

Die einzelnen Komponenten wurden kontinuierlich in einen Reaktor vom Typ List ORP 10 Kneter (List AG, Arisdorf, Schweiz) eindosiert. Der Durchsatz der Monomerlösung betrug 40 kg/h.

Die Reaktionslösung hatte am Zulauf eine Temperatur von ca. 23°C. Die Verweilzeit der Reaktionsmischung im Reaktor betrug ca. 15 Minuten. Nach Polymerisation und Gelzerkleinerung wurde jeweils 1 kg wässriges Polymergel auf Trockenblechen mit einem Boden aus 300µm Siebgewebe aufgegeben und im Umlufttrockenschrank 60 Minuten bei 175°C getrocknet. Das getrocknete Polymergel wurde mittels eines Walzenstuhles gemahlen und klassiert.

Zur Bestimmung der Farbstabilität wurden die Superabsorberpartikel 14 Tage bei 70°C und einer relativen Feuchte von 80% gelagert. Die Ergebnisse sind in der Tabelle 2 zusammengefasst.

### Beispiel 8

Es wurde verfahren wie unter Beispiel 7. Im ersten Drittel des Reaktors wurden zusätzlich 0,86 kg/h Superabsorber mit einer Partikelgröße kleiner 150 µm zugesetzt. Die zugesetzten Superabsorberpartikel waren thermisch oberflächennachvernetzt und enthielten kein Dinatriumsalz der 1-Hydroxyethyliden-1,1-diphosphonsäure.

Zur Bestimmung der Farbstabilität wurden die Superabsorberpartikel 14 Tage bei 70°C und einer relativen Feuchte von 80% gelagert. Die Ergebnisse sind in der Tabelle 2 zusammengefasst.

### Beispiel 9

Es wurde verfahren wie unter Beispiel 7. Im ersten Drittel des Reaktors wurden zusätzlich 1,72 kg/h Superabsorber mit einer Partikelgröße kleiner 150 µm zugesetzt. Die zugesetzten Superabsorberpartikel waren thermisch oberflächennachvernetzt und enthielten kein Dinatriumsalz der 1-Hydroxyethyliden-1,1-diphosphonsäure.

Zur Bestimmung der Farbstabilität wurden die Superabsorberpartikel 14 Tage bei 70°C und einer relativen Feuchte von 80% gelagert. Die Ergebnisse sind in der Tabelle 2 zusammengefasst.

### Beispiel 10

Es wurde verfahren wie unter Beispiel 7. Im ersten Drittel des Reaktors wurden zusätzlich 2,58 kg/h Superabsorber mit einer Partikelgröße kleiner 150 µm zugesetzt. Die zugesetzten Superabsorberpartikel waren thermisch oberflächennachvernetzt und enthielten kein Dinatriumsalz der 1-Hydroxyethyliden-1,1-diphosphonsäure.

Zur Bestimmung der Farbstabilität wurden die Superabsorberpartikel 14 Tage bei 70°C und einer relativen Feuchte von 80% gelagert. Die Ergebnisse sind in der Tabelle 2 zusammengefasst.

### Beispiel 11

Es wurde verfahren wie unter Beispiel 7. Im ersten Drittel des Reaktors wurden zusätzlich 3,44 kg/h Superabsorber mit einer Partikelgröße kleiner 150 µm zugesetzt. Die zugesetzten Superabsorberpartikel waren thermisch oberflächennachvernetzt und enthielten kein Dinatriumsalz der 1-Hydroxyethyliden-1,1-diphosphonsäure.

Zur Bestimmung der Farbstabilität wurden die Superabsorberpartikel 14 Tage bei 70°C und einer relativen Feuchte von 80% gelagert. Die Ergebnisse sind in der Tabelle 2 zusammengefasst.

**Tab. 2:**

| Bsp. | zugesetzter Superabsorber (berechnet auf hergestelltes Polymer) | Verfärbungen |
|---|---|---|
| 7 | ohne | + |
| 8 | 5 Gew.-% | - |
| 9 | 10 Gew.-% | - |
| 10 | 15 Gew.-% | -- |
| 11 | 20 Gew.-% | --- |

| | |
|---|---|
| keine sichtbaren punktuellen Verfärbungen | + |
| wenige sichtbare punktuelle Verfärbungen | - |
| deutliche sichtbare punktuelle Verfärbungen | -- |
| sehr deutliche sichtbare punktuelle Verfärbungen | --- |

Die Ergebnisse der Beispiele 7 bis 11 zeigen, dass die thermisch oberflächennachvernetzten Superabsorberpartikel kein Dinatriumsalz der 1-Hydroxyethyliden-1,1-diphosphonsäure mehr aufnehmen und sich während der Lagerung verfärben. Die punktuellen Verfärbungen nehmen mit der Menge an zugesetztem Superabsorber zu.

## Patentansprüche

1. Verfahren zur Herstellung von Superabsorberpartikeln durch Polymerisation einer Monomerlösung oder -suspension, enthaltend
a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
umfassend die Schritte
i) Polymerisation der Monomerlösung zu einem Polymergel,
ii) optional Zerkleinerung des erhaltenen Polymergels,
iii) Trocknung des Polymergels,
iv) Mahlung und Klassierung des getrockneten Polymergels zu Polymerpartikeln,
v) thermischer Oberflächennachvernetzung der klassierten Polymerpartikel,
vi) optional erneute Klassierung der oberflächennachvernetzten Polymerpartikel,
vii) optional Rückführung in Schritt iv) abgetrennter Polymerpartikel vor Schritt iii) und
viii) optional Rückführung in Schritt vi) abgetrennter Polymerpartikel vor Schritt iii),
**dadurch gekennzeichnet, dass** der Monomerlösung vor Schritt i) mindestens 0,75 Gew.-% einer Hydroxyphosphonsäure oder deren Salze, berechnet auf die Gesamtmenge an eingesetztem Monomer a), zugesetzt werden, dass den Polymerpartikeln zwischen Schritt iv) und Schritt vi) mindestens 0,09 Gew.-% Aluminiumkationen, berechnet auf die Gesamtmenge an eingesetzten Polymerpartikeln, zugesetzt werden und dass das Aluminiumkation als Aluminiumsulfat eingesetzt wird.

2. Verfahren nach Anspruch 1, **dadurch gekennzeichnet, dass** die in Schritt vi) abgetrennten und vor Schritt iii) rückgeführten Polymerpartikel eine Partikelgröße von weniger als 150 µm aufweisen.

3. Verfahren nach Anspruch 1 oder 2, **dadurch gekennzeichnet, dass** 1-Hydroxyethyliden-1,1'-diphosphonsäure als Hydroxyphosphonsäure eingesetzt wird.

4. Verfahren nach einem der Ansprüche 1 bis 3, **dadurch gekennzeichnet, dass** die Aluminiumkationen vor Schritt v) zugesetzt werden.

5. Verfahren nach einem der Ansprüche 1 bis 4, **dadurch gekennzeichnet, dass** der Monomerlösung vor Schritt i) mindestens 0,8 Gew.-% einer Hydroxyphosphonsäure oder deren Salze, berechnet auf die Gesamtmenge an eingesetztem Monomer a), zugesetzt werden.

6. Verfahren nach einem der Ansprüche 1 bis 4, **dadurch gekennzeichnet, dass** der Monomerlösung vor Schritt i) mindestens 0,85 Gew.-% einer Hydroxyphosphonsäure oder deren Salze, berechnet auf die Gesamtmenge an eingesetztem Monomer a), zugesetzt werden.

7. Verfahren nach einem der Ansprüche 1 bis 5, **dadurch gekennzeichnet, dass** den Polymerpartikeln nach Schritt iv) mindestens 0,1 Gew.-% Aluminiumkationen, berechnet auf die Gesamtmenge an eingesetzten Polymerpartikeln, zugesetzt werden.

8. Verfahren nach einem der Ansprüche 1 bis 6, **dadurch gekennzeichnet, dass** den Polymerpartikeln nach Schritt iv) mindestens 0,11 Gew.-% Aluminiumkationen, berechnet auf die Gesamtmenge an eingesetzten Polymerpartikeln, zugesetzt werden.

9. Superabsorberpartikel, erhältlich nach einem Verfahren der Ansprüche 1 bis 9, wobei die Superabsorberpartikel eine Zentrifugenretentionskapazität von mindestens 25 g/g, eine Absorption unter einem Druck von 49,2 g/cm² von mindestens 15 g/g, eine Gelbettpermeabilität von mindestens 60 Darcies aufweisen und die Superabsorberpartikel nach einer Lagerung von 14 Tagen bei einer Temperatur von 70°C und einer relativen Feuchte von 80% einen L-Wert von mindestens 67 einen a-Wert von weniger als 7,0 und einen b-Wert von weniger als 14,5 aufweisen, wobei die Zentrifugenretentionskapaziät, die Absorption unter einem Druck von 49,2 g/cm², die Gelbettpermeabilität, der L-Wert, der a-Wert und der b-Wert gemäß der in der Beschreibung im Abschnitt "Methoden" genannten Methoden bestimmt werden.

10. Superabsorberpartikel nach Anspruch 9, wobei die Zentrifugenretentionskapazität mindestens 28 g/g beträgt.

11. Superabsorberpartikel nach Anspruch 9 oder 10, wobei die Absorption unter einem Druck von 49,2 g/cm² mindestens 18 g/g beträgt.

12. Superabsorberpartikel nach einem der Ansprüche 9 bis 11, wobei die Gelbettpermeabilität mindestens 75 Darcies beträgt.

13. Superabsorberpartikel nach einem der Ansprüche 9 bis 12, wobei die Superabsorberpartikel nach einer Lagerung von 14 Tagen bei einer Temperatur von 70°C und einer relativen Feuchte von 80% einen L-Wert von mindestens 70 aufweisen.

14. Superabsorberpartikel nach einem der Ansprüche 9 bis 13, wobei die Superabsorberpartikel nach einer Lagerung von 14 Tagen bei einer Temperatur von 70°C und einer relativen Feuchte von 80% einen a-Wert von weniger als 5,8 und einen b-Wert von weniger als 13,0 aufweisen.

## Claims

1. A process for producing superabsorbent particles by polymerizing a monomer solution or suspension comprising
a) at least one ethylenically unsaturated monomer which bears acid groups and may have been at least partly neutralized,
b) at least one crosslinker,
c) at least one initiator,
d) optionally one or more ethylenically unsaturated monomers copolymerizable with the monomers mentioned under a) and
e) optionally one or more water-soluble polymers,
comprising the steps of
i) polymerizing the monomer solution to give a polymer gel,
ii) optionally comminuting the resulting polymer gel,
iii) drying the polymer gel,
iv) grinding and classifying the dried polymer gel to give polymer particles,
v) thermally surface postcrosslinking the classified polymer particles,
vi) optionally reclassifying the surface postcrosslinked polymer particles,
vii) optionally recycling polymer particles removed in step iv) upstream of step iii) and
viii) optionally recycling polymer particles removed in step vi) upstream of step iii),
which comprises adding to the monomer solution prior to step i) at least 0.75% by weight of a hydroxyphosphonic acid or salts thereof, calculated on the basis of the total amount of monomer a) used, and adding to the polymer particles between step iv) and step vi) at least 0.09% by weight of aluminum cations, calculated on the basis of the total amount of polymer particles used, and using the aluminum cation in the form of aluminum sulfate.

2. The process according to claim 1, wherein the polymer particles removed in step vi) and recycled upstream of step iii) have a particle size of less than 150 µm.

3. The process according to claim 1 or 2, wherein 1-hydroxyethylidene-1,1'-diphosphonic acid is used as hydroxyphosphonic acid.

4. The process according to any of claims 1 to 3, wherein the aluminum cations are added prior to step v) .

5. The process according to any of claims 1 to 4, wherein at least 0.8% by weight of a hydroxyphosphonic acid or salts thereof, calculated on the basis of the total amount of monomer a) used, is added to the monomer solution prior to step i).

6. The process according to any of claims 1 to 4, wherein at least 0.85% by weight of a hydroxyphosphonic acid or salts thereof, calculated on the basis of the total amount of monomer a) used, is added to the monomer solution prior to step i).

7. The process according to any of claims 1 to 5, wherein at least 0.1% by weight of aluminum cations, calculated on the basis of the total amount of polymer particles used, is added to the polymer particles after step iv).

8. The process according to any of claims 1 to 6, wherein at least 0.11% by weight of aluminum cations, calculated on the basis of the total amount of polymer particles used, is added to the polymer particles after step iv).

9. Superabsorbent particles obtainable by a process of claims 1 to 9, wherein the superabsorbent particles have a centrifuge retention capacity of at least 25 g/g, an absorption under a pressure of 49.2 g/cm² of at least 15 g/g, a gel bed permeability of at least 60 darcies, and the superabsorbent particles, after storage at a temperature of 70°C and a relative humidity of 80% for 14 days, have an L value of at least 67, an a value of less than 7.0 and a b value of less than 14.5, wherein the centrifuge retention capacity, absorption under a pressure of 49.2 g/cm², gel bed permeability, L value, a value and b value are determined by the method specified in the description in the "Methods" section.

10. Superabsorbent particles according to claim 9, wherein the centrifuge retention capacity is at least 28 g/g.

11. Superabsorbent particles according to claim 9 or 10, wherein the absorption under a pressure of 49.2 g/cm² is at least 18 g/g.

12. Superabsorbent particles according to any of claims 9 to 11, wherein the gel bed permeability is at least 75 darcies.

13. Superabsorbent particles according to any of claims 9 to 12, wherein the superabsorbent particles after storage at a temperature of 70°C and a relative humidity of 80% for 14 days have an L value of at least 70.

14. Superabsorbent particles according to any of claims 9 to 13, wherein the superabsorbent particles after storage at a temperature of 70°C and a relative humidity of 80% for 14 days have an a value of less than 5.8 and a b value of less than 13.0.

## Revendications

1. Procédé de fabrication de particules de superabsorbant par polymérisation d'une solution ou suspension de monomères, contenant :
a) au moins un monomère éthyléniquement insaturé, portant des groupes acides, qui peut être au moins partiellement neutralisé,
b) au moins un agent de réticulation,
c) au moins un initiateur,
d) éventuellement un ou plusieurs monomères éthyléniquement insaturés copolymérisables avec les monomères indiqués en a), et
e) éventuellement un ou plusieurs polymères solubles dans l'eau,
comprenant les étapes suivantes :
i) la polymérisation de la solution de monomères en un gel de polymère,
ii) éventuellement la fragmentation du gel de polymère obtenu,
iii) le séchage du gel de polymère,
iv) le broyage et la classification du gel de polymère séché en particules de polymère,
v) la post-réticulation en surface thermique des particules de polymère classifiées,
vi) éventuellement la nouvelle classification des particules de polymère post-réticulées en surface,
vii) éventuellement le recyclage de particules de polymère séparées dans l'étape iv) avant l'étape iii), et
viii) éventuellement le recyclage de particules de polymère séparées dans l'étape vi) avant l'étape iii),
**caractérisé en ce qu'**au moins 0,75 % en poids d'un acide hydroxyphosphonique ou ses sels, calculé par rapport à la quantité totale de monomère a) utilisé, sont ajoutés à la solution de monomères avant l'étape i), **en ce qu'**au moins 0,09 % en poids de cations aluminium, calculé par rapport à la quantité totale des particules de polymère utilisées, sont ajoutés aux particules de polymère entre l'étape iv) et l'étape vi), et **en ce que** le cation aluminium est utilisé sous la forme de sulfate d'aluminium.

2. Procédé selon la revendication 1, **caractérisé en ce que** les particules de polymère séparées dans l'étape vi) et recyclées avant l'étape iii) présentent une taille de particule de moins de 150 µm.

3. Procédé selon la revendication 1 ou 2, **caractérisé en ce que** de l'acide 1-hydroxyéthylidène-1,1'-diphosphonique est utilisé en tant qu'acide hydroxyphosphonique.

4. Procédé selon l'une quelconque des revendications 1 à 3, **caractérisé en ce que** les cations aluminium sont ajoutés avant l'étape v).

5. Procédé selon l'une quelconque des revendications 1 à 4, **caractérisé en ce qu'**au moins 0,8 % en poids d'un acide hydroxyphosphonique ou ses sels, calculé par rapport à la quantité totale de monomère a) utilisé, sont ajoutés à la solution de monomères avant l'étape i).

6. Procédé selon l'une quelconque des revendications 1 à 4, **caractérisé en ce qu'**au moins 0,85 % en poids d'un acide hydroxyphosphonique ou ses sels, calculé par rapport à la quantité totale de monomère a) utilisé, sont ajoutés à la solution de monomères avant l'étape i).

7. Procédé selon l'une quelconque des revendications 1 à 5, **caractérisé en ce qu'**au moins 0,1 % en poids de cations aluminium, par rapport à la quantité totale de particules de polymère utilisées, sont ajoutés aux particules de polymère après l'étape iv).

8. Procédé selon l'une quelconque des revendications 1 à 6, **caractérisé en ce qu'**au moins 0,11 % en poids de cations aluminium, par rapport à la quantité totale de particules de polymère utilisées, sont ajoutés aux particules de polymère après l'étape iv).

9. Particules de superabsorbant, pouvant être obtenues par un procédé selon les revendications 1 à 9, les particules de superabsorbant présentant une capacité de rétention centrifuge d'au moins 25 g/g, une absorption sous une pression de 49,2 g/cm² d'au moins 15 g/g, une perméabilité en lit de gel d'au moins 60 Darcies, et les particules de superabsorbant présentant après un stockage de 14 jours à une température de 70 °C et une humidité relative de 80 % une valeur L d'au moins 67, une valeur a de moins de 7,0 et une valeur b de moins de 14,5, la capacité de rétention centrifuge, l'absorption sous une pression de 49,2 g/cm², la perméabilité en lit de gel, la valeur L, la valeur a et la valeur b étant déterminées selon les méthodes indiquées dans la description dans la section « méthodes ».

10. Particules de superabsorbant selon la revendication 9, dans lesquelles la capacité de rétention centrifuge est d'au moins 28 g/g.

11. Particules de superabsorbant selon la revendication 9 ou 10, dans lesquelles l'absorption sous une pression de 49,2 g/cm² est d'au moins 18 g/g.

12. Particules de superabsorbant selon l'une quelconque des revendications 9 à 11, dans lesquelles la perméabilité en lit de gel est d'au moins 75 Darcies.

13. Particules de superabsorbant selon l'une quelconque des revendications 9 à 12, les particules de superabsorbant présentant après un stockage de 14 jours à une température de 70 °C et une humidité relative de 80 % une valeur L d'au moins 70.

14. Particules de superabsorbant selon l'une quelconque des revendications 9 à 13, les particules de superabsorbant présentant après un stockage de 14 jours à une température de 70 °C et une humidité relative de 80 % une valeur a de moins de 5,8 et une valeur b de moins de 13,0.
